# EUROPEAN PATENT APPLICATION

(11) **EP 3 605 153 A1**
(43) Date of publication of application: **05.02.2020**
(21) Application number: 18775984.0
(22) Date of filing: 20.03.2018
(51) Int. Cl.: G01V 9/00, A01M 1/00, G01N 5/02, G01N 27/12

(54) **INSECT DETECTION METHOD, GAS SENSOR FOR INSECT DETECTION, GAS SENSOR ARRAY FOR INSECT DETECTION, AND ELECTRIC MACHINE PRODUCT**

(30) Priority: 28.03.2017 JP 2017063606
(71) Applicant: FUJIFILM Corporation, Tokyo 106-8620 (JP)
(72) Inventor: AIKI, Yasuhiro, Ashigarakami-gun, Kanagawa 258-8577 (JP); SANO, Takahiro, Ashigarakami-gun, Kanagawa 258-8577 (JP); MOCHIZUKI, Fumihiko, Ashigarakami-gun, Kanagawa 258-8577 (JP); TAKAKU, Koji, Ashigarakami-gun, Kanagawa 258-8577 (JP)
(74) Representative: Klunker IP Patentanwälte PartG mbB
(86) International application number: PCT/JP2018/011088
(87) International publication number: WO 2018/180792

(57) **Abstract**

Provided are an insect detection method that includes detecting an intrinsic gas emitted by an insect using a gas sensor including a gas adsorption membrane, the gas sensor being selected from a resonant sensor, an electrical resistance sensor, and a field effect transistor sensor; a gas sensor for insect detection and a gas sensor array for insect detection, which are suitable to be used for this method; and an electric machine product having the gas sensor or gas sensor array mounted therein.

## Description

### BACKGROUND OF THE INVENTION

### 1. Field of the Invention

The present invention relates to an insect detection method, a gas sensor for insect detection, a gas sensor array for insect detection, and an electric machine product.

### 2. Description of the Related Art

In advanced countries, problems of allergic diseases such as asthma and atopic diseases have emerged to the surface. Mites are found throughout the advanced countries, and it is known that dead bodies of mites or pheromones or the like released by mites induce allergic diseases. Therefore, it is a very important task in hygiene to figure out the population of mites and remove mites.

Furthermore, cockroaches, termites, aphids, and the like are known as insect pests that inhabit the human residence area.

It is known that insects release a variety of pheromones into air, and these pheromones act between individual of the same kind and affect the behavior of the insects. Several technologies for specifically detecting the pheromones of insects have been reported, and for example, it is described in JP2012-078351A that in a case in which the pheromone receptors of Drosophila melanogaster are expressed in bombykol receptor cells of silkworm moth, this silkworm moth can be utilized as a pheromone detection sensor for Drosophila melanogaster.

It is also described in JP2013-027376A that olfactory cells of an insect, which stably express an olfactory receptor protein of the insect for an odor substance as an object of detection, receive the odor substance, and then emit light, has been established, and that these cells function as an insect pheromone sensor.

### SUMMARY OF THE INVENTION

Mites are generally small, and the presence thereof cannot be checked by visual inspection. Many of insect pests such as cockroaches inhabit those out-of-sight places, and it is difficult to visually check the presence, population, and the like.

In order to comprehend the presence of these insects, it can be considered to detect the intrinsic gases such as pheromones emitted by the insects. However, since the detection of pheromones by biosensors that use genetic engineering techniques as described above uses living organisms or cells, the productivity is low, consequently the cost is high, and it cannot be said to be satisfactory even in view of the measurement accuracy and reproducibility.

Thus, it is an object of the present invention to provide a method by which the presence of insects can be detected conveniently at lower cost with higher accuracy. It is another object of the invention to provide a gas sensor for insect detection or a gas sensor array for insect detection, which is suitable for carrying out the method described above, and an electric machine product having these mounted therein.

The above-described problems of the invention were solved by the following means.
[1] An insect detection method, comprising detecting a characteristic gas emitted by an insect using a gas sensor including a gas adsorption membrane, the gas sensor being selected from a resonant sensor, an electrical resistance sensor, and a field effect transistor sensor.
[2] The insect detection method according to [1], wherein a dielectric material carried by the resonant sensor is a ceramic dielectric material or a quartz crystal.
[3] The insect detection method according to [2], wherein the ceramic dielectric material is lead zirconate titanate, niobium-doped lead zirconate titanate, zinc oxide, or aluminum nitride.
[4] The insect detection method according to any one of [1] to [3], wherein the method includes detecting an intrinsic gas emitted by an insect using a plurality of gas sensors, and the plurality of gas sensors have mutually different gas responsiveness.
[5] The insect detection method according to [4], wherein an insect is detected on the basis of a pattern of gas detection signals by the plurality of gas sensors.
[6] The insect detection method according to any one of [1] to [5], wherein the gas sensor is a resonant sensor.
[7] The insect detection method according to any one of [1] to [6], wherein the insect is a mite, a cockroach, a termite, or an aphid.
[8] The insect detection method according to [7], wherein the insect is a mite.
[9] A gas sensor for insect detection, comprising a gas adsorption membrane, the gas sensor being selected from a resonant sensor, an electrical resistance sensor, and a field effect transistor sensor,
   wherein the gas adsorbing material that constitutes the gas adsorption membrane of the gas sensor has a solubility parameter of 13.9 to 23.9.
[10] A gas sensor array for insect detection, comprising a plurality of the gas sensors for insect detection according to [9] integrated together, wherein this plurality of gas sensors have mutually different gas responsiveness.
[11] An electric machine product, comprising the gas sensor for insect detection according to [9] or the gas sensor array for insect detection according to [10] mounted therein.
[12] The electric machine product according to [11], wherein the electric machine product is a vacuum cleaner.

According to the present specification, a numerical value range indicated using the symbol "∼" means a range including the numerical values described before and after the symbol "∼" as the lower limit and the upper limit.

According to the insect detection method of the invention, the presence of a particular insect can be conveniently detected at lower cost with higher accuracy. The gas sensor for insect detection or the gas sensor array for insect detection according to the invention is a device suitable for carrying out the above-described detection method. The electric machine product according to the invention comprises the gas sensor for insect detection or the gas sensor array for insect detection according to the invention mounted therein, and has a function of detecting a particular insect.

### BRIEF DESCRIPTION OF THE DRAWINGS

Fig. 1 is a cross-sectional view schematically illustrating an example of a resonant sensor.
Fig. 2 is a cross-sectional view schematically illustrating an example of a field effect transistor sensor.
Fig. 3A is a cross-sectional view schematically illustrating an SOI substrate of a gas sensor produced in Examples.
Fig. 3B is a cross-sectional view schematically illustrating a state in which, in the production flow for the gas sensor produced in Examples, a first electrode is provided on the SOI substrate, and a PZTN film is provided on the first electrode.
Fig. 3C is a cross-sectional view schematically illustrating a state in which, in the production flow for the gas sensor produced in Examples, resist patterning is performed on the PZTN film.
Fig. 3D is a cross-sectional view schematically illustrating a state in which, in the production flow for the gas sensor produced in Examples, the PZTN film is wet-etched.
Fig. 3E is a cross-sectional view schematically illustrating a state in which, in the production flow for the gas sensor produced in Examples, after the PZTN film has been wet-etched, the resist is removed using a resist stripping liquid.
Fig. 3F is a cross-sectional view schematically illustrating a state in which, in the production flow for the gas sensor produced in Examples, resist patterning for forming a contact with the first electrode and resist patterning for forming a second electrode is carried out.
Fig. 3G is a cross-sectional view schematically illustrating a state in which, in the production flow for the gas sensor produced in Examples, a contact with the first second, and a second electrode are formed.
Fig. 3H is a cross-sectional view schematically illustrating a state in which, in the production flow for the gas sensor produced in Examples, the resist has been removed using a resist stripping liquid after a contact with the first electrode and a second electrode have been formed.
Fig. 3I is a cross-sectional view schematically illustrating a state in which, in the production flow for the gas sensor produced in Examples, resist patterning for patterning of the first electrode and the substrate surface layer (15-µm Si film) is carried out.
Fig. 3J is a cross sectional view schematically illustrating a state in which, in the production flow for the gas sensor produced in Examples, a portion of the first electrode is removed by dry etching.
Fig. 3K is a cross-sectional view schematically illustrating a state in which, in the production flow for the gas sensor produced in Examples, a portion of the Si film is removed by etching.
Fig. 3L is a cross-sectional view schematically illustrating a state in which, in the production flow for the gas sensor produced in Examples, after a portion of the Si film has been removed by etching, the resist is removed using a resist stripping liquid.
Fig. 3M is a cross-sectional view schematically illustrating a state in which, in the production flow for the gas sensor produced in Examples, a photoresist is formed on the second electrode side for the purpose of protection in the subsequent process.
Fig. 3N is a cross-sectional view schematically illustrating a state in which, in the production flow for the gas sensor produced in Examples, resist patterning for patterning of the lower surface of the substrate is carried out.
Fig. 3O is a cross-sectional view schematically illustrating a state in which, in the production flow for the gas sensor produced in Examples, A SiO₂ film is removed by dry etching from the lower surface of the substrate.
Fig. 3P is a cross-sectional view schematically illustrating a state in which, in the production flow for the gas sensor produced in Examples, a Si film is removed by dry etching from the lower surface of the substrate.
Fig. 3Q is a cross-sectional view schematically illustrating a state in which, in the production flow for the gas sensor produced in Examples, a SiO₂ film is removed by dry etching from the lower surface of the substrate.
Fig. 3R is a cross-sectional view schematically illustrating a state in which, in the production flow for the gas sensor produced in Examples, after a SiO₂ film has been removed by drying etching from the lower surface of the substrate, the entire resist is removed using a resist stripping liquid.
Fig. 3S is a cross-sectional view schematically illustrating a state in which, in the production flow for the gas sensor produced in Examples, a gas adsorption membrane is formed on the second electrode.
Fig. 4 is a graph showing the results of detecting mites with a gas sensor array in Examples.

### DESCRIPTION OF THE PREFERRED EMBODIMENTS

Preferred embodiments of the invention will be described below; however, the invention is not intended to be limited to these embodiments.

### [Insect detection method]

The insect detection method according to the embodiment of the invention (hereinafter, also simply referred to as "detection method according to the embodiment of the invention") includes detecting an intrinsic gas such as pheromones emitted by an insect using a gas sensor selected from a resonant sensor, an electrical resistance sensor, and a field effect transistor sensor. All of these sensors have a gas adsorption membrane that adsorbs a desired gas emitted by an insect, and detect a change in the signal (change in the resonant frequency, electrical resistance, or transistor characteristics) caused by a gas adsorbed to this gas adsorption membrane.

By using the gas sensor described above, an intrinsic gas emitted by an insect can be detected more reliably with higher accuracy, and as a result, the presence of an insect emitting a gas can be detected with higher accuracy.

### <Resonant sensor>

A resonant sensor adsorbs gas molecules of a particular species included in air to the surface, comprehends the presence or absence of adsorption or the amount of adsorption as the amount of decrease in the resonant frequency of a dielectric material (piezoelectric material) to be resonance-driven, and detects a target gas. That is, a resonant sensor is a sensor that utilizes a mass micro-balancing method.

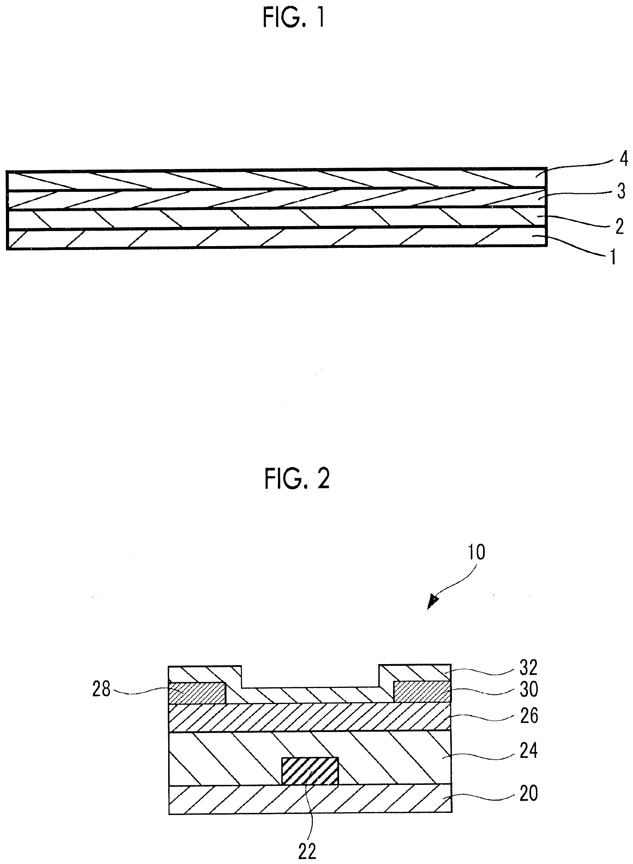

Fig. 1 is a cross-sectional view schematically illustrating a laminated structure according to an embodiment of the resonant sensor used for the embodiments of the invention. The resonant sensor illustrated in Fig. 1 has a laminated structure in which a first electrode 1, a dielectric material 2, a second electrode 3, and a gas adsorption membrane 4 are provided in sequence. On a surface of the first electrode, the surface being on the opposite side of the side that is in contact with the dielectric material 2, a substrate for supporting the resonant sensor may be provided. In a case in which the dielectric material is of self-excited oscillation type, the substrate is not essential. On the other hand, in a case in which the dielectric material is a ceramic piezoelectric element or the like, a substrate is needed for resonance-driving the element.

In sensing according to a mass micro-balancing method, a voltage is applied to a fine dielectric material (piezoelectric material), thereby the dielectric material is oscillated at a constant frequency (resonant frequency), and an increase in mass caused by gas adsorption to the dielectric material surface is detected as a reduction in the resonant frequency. As a representative example of a sensor that utilizes the mass micro-balancing method, a QCM (Quartz Crystal Mass micro-balancing) sensor that uses a quartz oscillator as a dielectric material for resonance-driving is known.

In a QCM sensor, usually, an electrode is provided on both surfaces of a thin film of quartz crystal cut at a particular angle (AT-cut), a voltage is applied, and thereby the sensor is subjected to shear oscillation at a resonant frequency in a direction horizontal to the crystal face. Since this resonant frequency decreases according to the mass of the gas adsorbed onto the electrodes, a change in the mass of a substance on the electrode can be recognized. A QCM sensor having a crystal oscillator and electrodes disposed so as to interpose this oscillator is known per se and can be produced by conventional methods. A commercially available product may also be used.

The QCM sensor used for the embodiments of the invention has, in order to adsorb a gas onto the electrodes, a gas adsorption membrane containing a gas adsorbing material on the surface of one electrode between a pair of electrodes provided so as to interpose a dielectric material. The mass of the gas adsorbed to this gas adsorption membrane is detected as a decrease in the resonant frequency of the crystal oscillator that is resonance-driven. The gas adsorbing material will be described later.

The electrodes used for the resonant sensor are not particularly limited, and any metal material or the like that is conventionally used as an electrode can be used.

As the resonant sensor, in addition to the QCM sensor, a resonant sensor that does not use quartz crystal, quartz, or the like as the dielectric material but uses a ceramic dielectric substance (piezoelectric material), can be employed. Examples of such a sensor include a cantilever type sensor and a surface acoustic wave (SAW) sensor. Since a ceramic dielectric material can be formed into a film on a substrate by vacuum vapor deposition or the like, a ceramic dielectric material has an advantage that it can be applied to the production of a sensor using the MEMS (Micro Electro Mechanical Systems) technology. Examples of such a ceramic dielectric material include lead zirconate titanate (PZT), niobium-doped lead zirconate titanate (PZTN), zinc oxide (ZnO), and aluminum nitride (AIN).

In a cantilever type sensor, an electrode is disposed on both surfaces of a film formed from the above-described ceramic dielectric material, and the ceramic delectric material can be resonance-driven by applying a particular voltage between the electrodes. The resonant sensor that uses a ceramic dielectric material, which is used for the gas sensor according to the embodiment of the invention, also has a gas adsorption membrane containing a gas adsorbing material on the surface of one electrode between a pair of electrodes provided so as to interpose a dielectric material, in order to adsorb a gas onto the electrodes. The mass of the gas adsorbed to this gas adsorption membrane is detected as a decrease in the resonant frequency of the ceramic dielectric material that is resonance-driven. The gas adsorbing material will be described later.

A resonant sensor that uses a ceramic dielectric material can be produced by, for example, the method described in the Examples that will be described later.

### <Electrical resistance sensor>

In an electrical resistance sensor used for the embodiments of the invention, an electrically conductive gas adsorption membrane is connected between electrodes provided on a substrate, a voltage is applied between the electrodes, and the amount of adsorption of a gas to the gas adsorption membrane is recognized through an increase in the electrical resistance between the electrodes. The configuration of an electrical resistance sensor is known per se, and for example, paragraphs [0023] to [0028] of JP2002-526769A can be referred to.

In regard to the electrical resistance sensor used for the embodiments of the invention, the gas adsorption membrane that connects between two electrodes is required to have electrical conductivity. Therefore, in a case in which the gas adsorbing material constituting the gas adsorption membrane is an insulating material, an electroconductive material is incorporated into the gas adsorption membrane, together with the gas adsorbing material that will be described later. On the other hand, in a case in which the gas adsorbing material is electroconductive, the gas adsorption membrane may be composed of a gas adsorbing material. The gas adsorbing material will be described later.

The electroconductive material may be an organic electroconductive material, may be an inorganic electroconductive material, or may be an inorganic/organic mixed electroconductive material. Specific examples of these electroconductive materials include, for example, the materials described in [Table 2] of paragraph [0028] of JP2002-526769A.

Regarding the substrate for the electrical resistance sensor, an insulating substrate is preferred, and for example, a glass substrate can be used.

There are no particular limitations on the electrodes used for the electrical resistance sensor, and any metal material or the like that is conventionally used as an electrode can be used.

### <Field effect transistor sensor>

In a field effect transistor (FET) sensor used for the embodiments of the invention, a gas adsorption membrane containing a gas adsorbing material is provided to be in contact with a semiconductor layer of a transistor, and adsorption of gas molecules to the gas adsorption membrane is recognized through an increase in the resistance of the semiconductor layer.

Fig. 2 is a cross-sectional view schematically illustrating a preferred embodiment of the FET sensor used for the embodiments of the invention. As shown in Fig. 2, a transistor 10 includes a substrate 20; a gate electrode 22 disposed on the substrate 20; a gate insulating layer 24 disposed so as to cover the gate electrode 22; a semiconductor layer 26 disposed on the gate insulating layer 24; a source electrode 28 and a drain electrode 30 disposed to be separated apart from each other on the semiconductor layer 26; and a gas adsorption membrane 32 disposed on the source electrode 28, drain electrode 30, and semiconductor layer 26, and containing a gas adsorbing material. The transistor 10 is a so-called bottom gate-top contact type transistor.

In the FET sensor, in a case in which gas molecules adsorb to the gas adsorption membrane, the electrical resistance of the semiconductor layer disposed adjacent to the gas adsorption membrane changes, and as a result, the electrical characteristics of the transistor are changed. By detecting this change in the electrical characteristics of the transistor, a gas can be detected.

The type of the change in the electrical characteristics of the transistor is not particularly limited, and examples include a change in the current value between the source electrode and the drain electrode (current value of the drain current), a change in the carrier mobility, and a change in voltage. Among them, from the viewpoint that measurement is easy, it is preferable to detect a change in the current value between the source electrode and the drain electrode (current value of the drain current).

The various members constituting the FET sensor used for the embodiments of the invention will be described; however, the invention is not intended to be limited to the following embodiments. The gas adsorbing material constituting the gas adsorption membrane will be described later because the gas adsorbing material is used in common with other sensors.

### - Substrate -

A substrate 20 is a base material that supports various members such as a gate electrode 22.

The type of the substrate 20 is not particularly limited, and mainly, glass or a plastic film can be used. There are no particular limitations on the plastic film, and for example, films formed from polyethylene terephthalate (PET), polyethylene naphthalate (PEN), polyether ether ketone, polyphenylene sulfide, polyallylate, polyimide, polycarbonate (PC), cellulose triacetate (TAC), and cellulose acetate propionate (CAP) can be used.

In a case in which the gate electrode 22 that will be described later functions also as a substrate, it is acceptable not to provide the substrate 20.

### - Gate electrode -

A gate electrode 22 is an electrode disposed on the substrate 20.

The material constituting the gate electrode 22 is not particularly limited as long as it is an electroconductive material, and examples include metals such as gold (Au), silver, aluminum (Al), copper, chromium, nickel, cobalt, titanium, platinum, magnesium, calcium, barium, and sodium; electroconductive oxides such as InO₂, SnO₂, and ITO; electroconductive polymers such as polyaniline, polypyrrole, polythiophene, polyacetylene, and polydiacetylene; semiconductors such as silicon, germanium, and gallium arsenide; and carbon materials such as fullerene, carbon nanotubes, and graphite. Among them, the material constitutes the gate electrode is preferably a metal, and more preferably silver or aluminum.

The thickness of the gate electrode 22 is not particularly limited; however, the thickness is preferably 20 to 1,000 nm.

The pattern shape of the gate electrode 22 is not particularly limited, and any optimal shape is selected as appropriate.

The method for forming the gate electrode 22 is not particularly limited. For example, an electroconductive thin film formed on the substrate 20 by vapor deposition, sputtering or the like is subjected to an etching treatment or the like to form a gate electrode (22); or a mask having a predetermined pattern is disposed on the substrate 20, and a gate electrode 22 can be formed by vapor deposition, sputtering, or the like.

A gate electrode 22 may be formed by performing patterning directly on the substrate 20 by an inkjet method using a solution or a dispersion liquid of an electroconductive polymer, or the gate electrode 22 may also be formed from a coating film using a photolithography method or a laser ablation method. Furthermore, it is also acceptable to perform patterning by a printing method such as letterpress printing, intaglio printing, lithography, or screen printing, using an ink containing an electroconductive polymer or electroconductive microparticles, an electroconductive paste, or the like.

### - Gate insulating layer -

A gate insulating layer 24 is a layer disposed on the substrate 20 so as to cover the gate electrode 22. Examples of the material of the gate insulating layer 24 include polymers such as polymethyl methacrylate, polystyrene, polyvinyl phenol, polyimide, polycarbonate, polyester, polyvinyl alcohol, polyvinyl acetate, polyurethane, polysulfone, polybenzoxazol, polysilsesquioxane, an epoxy resin, and a phenolic resin; oxides such as silicon dioxide, aluminum oxide, and titanium oxide; and nitrides such as silicon nitride. Among these materials, as the material for the gate insulating layer 24, it is preferable to use an organic insulating material from the viewpoint of handleability.

In a case in which a polymer is used as the material of the gate insulating layer 24, it is preferable to use a crosslinking agent (for example, melamine) in combination. The polymer is crosslinked by using a crosslinking agent in combination, and the durability of the formed gate insulating layer 24 is enhanced.

The thickness of the gate insulating layer 24 is not particularly limited, and the thickness is preferably 50 nm to 3 µm, and more preferably 200 nm to 1 µm.

The method for forming the gate insulating layer 24 is not particularly limited. For example, a method of applying a composition for gate insulating layer formation including an organic insulating material on a substrate 20 on which a gate electrode 22 has been formed, and thereby forming a gate insulating layer 24; and a method of forming a gate insulating layer 24 by vapor deposition or sputtering, may be mentioned.

The composition for gate insulating layer formation may include a solvent (water, or an organic solvent), as necessary. Furthermore, the composition for gate insulating layer formation may include a crosslinking component. For example, a crosslinked structure can be introduced into the gate insulating layer 24 by adding a crosslinking component such as melamine into an organic insulating material containing a hydroxy group.

The method of applying the composition for gate insulating layer formation is not particularly limited, and wet processes such as a method based on application, such as a spray coating method, a spin coating method, a blade coating method, a dip coating method, a casting method, a roll coating method, a bar coating method, or a die coating method; and a method based on patterning, such as inkjetting, are preferred.

In a case in which a gate insulating layer 24 is formed by applying a composition for gate insulating layer formation, the composition may be heated (baking) after application, for the purpose of solvent removal, crosslinking, and the like.

### - Semiconductor layer -

A semiconductor layer 26 is a layer disposed on the gate insulating layer 24, and is a layer in which the electrical characteristics (particularly, electrical resistance) change in a case in which adsorption of gas molecules to the gas adsorption membrane 32 occurs.

The material constituting the semiconductor layer 26 may be an organic semiconductor or an inorganic semiconductor, and from the viewpoint of having excellent productivity, sensitivity, and the like, the material is preferably an organic semiconductor.

Examples of the organic semiconductor include pentacene compounds such as 6,13-bis(triisopropylsilylethynyl)pentacene (TIPS pentacene), tetramethylpentacene, and perfluoropentacene; anthradithiophenes such as TES-ADT and diF-TES-ADT (2,8-difluoro-5,11-bis(triethylsilylethynyl)anthradithiophene); benzothienobenzothiophenes such as DPh-BTBT and Cn-BTBT; dinaphthothienothiophenes such as Cn-DNTT; dioxaanthanthrenes such as perixanthenoxanthene; rubrenes; fullerenes such as C60 and PCBM; phthalocyanines such as copper phthalocyanine and fluorinated copper phthalocyanine; polythiophenes such as P3RT, PQT, P3HT, and PQT; and polythienothiophenes such as poly[2,5-bis(3-dodecylthiophen-2-yl)thieno[3,2-b]thiophene] (PBTTT).

Examples of the inorganic semiconductor include oxides of one kind or a mixture of two or more kinds from among indium (In), gallium (Ga), tin (Sn), zinc (Zn), and the like. A specific example may be indium gallium zinc oxide (IGZO, InGaZnO). In addition to indium gallium zinc oxide, an In-Al-Zn-O system, an In-Sn-Zn-O system, an In-Zn-O system, an In-Sn-O system, a Zn-O system, a Sn-O system, and the like may also be used.

The thickness of the semiconductor layer 26 is not particularly limited; however, the thickness is preferably 200 nm or less, and more preferably 50 nm or less. The lower limit is not particularly limited; however, the lower limit is 10 nm or more in many cases.

The method for forming the semiconductor layer 26 is not particularly limited; however, for example, a method of depositing a semiconductor compound on the gate insulating layer 24 by vapor deposition or sputtering, and forming a semiconductor layer 26 (dry method), and a method of applying a semiconductor composition including a semiconductor compound on the gate insulating layer 24, performing a drying treatment as necessary, and thereby forming a semiconductor layer 26 (wet method), may be mentioned.

### - Source electrode and drain electrode -

A source electrode 28 and a drain electrode 30 are electrodes that are disposed on the semiconductor layer 26 and are disposed to be separated apart from each other.

The source electrode 28 and the drain electrode 30 are rectangular-shaped electrodes that extend in directions orthogonal to the directions facing each other.

Regarding the materials constituting the source electrode 28 and the drain electrode 30, the materials constituting the gate electrode 22 described above may be mentioned. Regarding the method for forming the source electrode 28 and the drain electrode 30, the method for forming the gate electrode 22 described above may be mentioned.

The thicknesses of the source electrode 28 and the drain electrode 30 are not particularly limited; however, the thicknesses are preferably 20 to 1,000 nm.

The channel lengths of the source electrode 28 and the drain electrode 30 are not particularly limited; however, the channel lengths are preferably 5 to 30 µm.

The channel widths of the source electrode 28 and the drain electrode 30 are not particularly limited; however, the channel widths are preferably 10 to 200 µm.

### - Other layers -

The transistor 10 may include another layer other than the members described above.

For example, a self-assembled monomolecular film may be disposed between the gate insulating layer 24 and the semiconductor layer 26. A carrier injection layer may also be disposed between the semiconductor layer 26 and the source electrode 28 (or drain electrode 30).

### [Gas adsorption membrane]

In the various sensors of resonant type, electrical resistance type, and FET type as described above, the gas adsorption membrane contains a gas adsorbing material. The content of the gas adsorbing material in the gas adsorption membrane is preferably 20% by mass or more, more preferably 40% by mass or more, and even more preferably 60% by mass or more. It is also preferable that the gas adsorption membrane is a film formed from a gas adsorbing material.

This gas adsorbing component is not particularly limited as long as it has an adsorption ability for an intrinsic gas (pheromones or the like) emitted by an insect, and the gas adsorbing component is selected as appropriate according to the purpose. The gas adsorbing material may be an inorganic material or may be an organic material, and from the viewpoint of exhibiting adsorption ability for various organic gases, it is preferable that the gas adsorbing material is an organic material.

Examples of the organic material that can be used as the gas adsorbing material include polyvinyl stearate (16.14), polybutyl methacrylate (19.13), polymethylstyrene (19.96), polyethylene oxide (10.62), polyethylene chloride (20.95), polyethylene vinyl acetate (21.06), polytribromostyrene (21.49), polycaprolactone (21.67), polyvinylphenol (23.25), polyvinyl acetate (24.17), polyhydroxyethyl methacrylate (24.97), triacetyl cellulose (26.28), and polyvinylpyrrolidone (27.84). The values in the parentheses are solubility parameters (SP values).

In a case in which an organic material having an SP value that is close to the SP value of an intrinsic gas emitted by the insect as a target of detection is used for the gas adsorption membrane, the gas adsorptiveness tends to be further increased.

The SP value according to the invention is a value calculated by the Okitsu method. The Okitsu method is described in detail in, for example, Journal of the Adhesion Society of Japan, 1993, Vol. 29, No. 6, p. 249-259.

From the viewpoint of further increasing gas adsorptiveness, the absolute value of the difference between the SP value of the gas as a target of detection and the SP value of the gas adsorbing material is preferably 5.0 or less, and more preferably 3.0 or less. For example, in a case in which it is intended to detect geranial, which is one of the gases emitted by mites, since the SP value of geranial is 18.9, an organic polymer having an SP value of 13.9 to 23.9 can be used as the gas adsorbing material used for the gas adsorption membrane of the sensor, and it is preferable to use an organic polymer having an SP value of 15.9 to 21.9.

In the detection method according to the embodiment of the invention, it is preferable that an intrinsic gas emitted by an insect is detected using a plurality of gas sensors having mutually different responsiveness to the intrinsic gas emitted by the insect. That is, it is preferable that a plurality of gas sensors having mutually different responsiveness to an intrinsic gas emitted by an insect is arrayed (multi-channeling), and the insect is detected on the basis of the patterns of gas detection signals produced by the arrayed gas sensors (collection of signal patterns of a plurality of gas sensors). A plurality of gas sensors having mutually different responsiveness to an intrinsic gas emitted by an insect can be realized by the selection of gas adsorbing materials constituting the gas adsorption membranes carried by the sensors.

As such, it is made possible to detect an insect with higher accuracy through the detection of an insect based on the signal patterns produced by a plurality of sensors. For example, five gas sensors a, b, c, d, and e having different gas responsiveness to an intrinsic gas emitted by an insect are prepared, and the signal patterns of these five gas sensors obtainable at the time of being brought into contact with a gas emitted by a particular mite are investigated in advance (for example, in a case in which the signal intensities of the various gas sensors at the time of being brought into contact with a gas emitted by a particular mite are presented as relative values of five stages, signal intensity of a: 5, signal intensity of b: 4, signal intensity of c: 3, signal intensity of d: 2, signal intensity of e: 1, or the like). In a case in which a gas in a certain space is detected, the relative relationship between the signal intensities (signal patterns) of the five sensors is compared with the signal patterns that have been investigated in advance as described above, and in a case in which the signal patterns coincide, it can be considered that the particular mite exists in the space. By investigating the signal patterns related to multiple kinds of insects in advance and establishing a database, it is also possible to discriminate the kinds of insects existing in the space.

Detection of an insect based on the signal patterns of a plurality of sensors can be achieved as detection with higher accuracy even for the detection of an insect emitting a plurality of different gases. That is, by arraying a plurality of gas sensors having mutually different gas responsiveness, and based on the complex patterns of the signal intensities of the various sensors, a plurality of intrinsic gases emitted by an insect can be detected with higher accuracy, and it is also possible to discriminate the kind of the insect emitting this plurality of gases, or the like. Meanwhile, in the plurality of gas sensors constituting the gas sensor array, a gas sensor including a gas adsorption membrane having low adsorptiveness to the intrinsic gas emitted by a particular insect, or a gas sensor including a gas adsorption membrane that does not have adsorptiveness to the intrinsic gas emitted by a particular insect, may be incorporated. In this manner, the variation of the complex patterns of the signal intensities of the various sensors is increased, and thereby the accuracy of identification is increased. Furthermore, isolation of gas components other than the gases emitted by the insect can be carried out with higher accuracy.

According to the invention, in a case in which it is said that a plurality of gas sensors have mutually different gas responsiveness, it is implied to include an embodiment in which all of the plurality of gas sensors have mutually different gas responsiveness, and an embodiment in which some gas sensors among the plurality of gas sensors have the same gas responsiveness.

In a case in which an insect is detected using a plurality of gas sensors having mutually different responsiveness to an intrinsic gas emitted by the insect, the number of the gas sensors is preferably 2 to 100, more preferably 2 to 50, and even more preferably 4 to 30.

The gas sensors constituting the gas sensor array may include two or more kinds of sensors selected from a resonant sensor, an electrical resistance sensor, and an FET sensor; however, usually, all of the gas sensors constituting the gas sensor array are resonant sensors, all are electrical resistance sensors, or all are FET sensors. More preferably, all of the gas sensors constituting the gas sensor array are resonant sensors.

In a case in which the sensors are arrayed as described above, size reduction of the sensors is required. From this point of view, it is preferable to employ resonant sensors that use a ceramic dielectric material, which can be produced by MEMS technology.

### <Detection target gas>

In regard to the detection method according to the embodiment of the invention, the insect as a target of detection is not particularly limited as long as it is an insect that emits gases such as pheromones. From the viewpoint of detecting so-called insect pests in the residence area, it is preferable that the target of detection is a mite, a cockroach, a termite, or an aphid, and it is more preferable that the target of detection is a mite. Examples of the intrinsic gas emitted by these insects will be listed below.

### - Trail pheromone of termite -

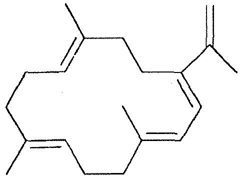

### - Ranking pheromones of Reticulitermes -

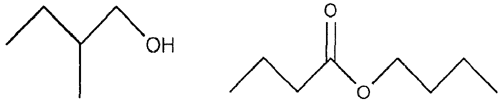

### - Sex pheromone of Periplaneta fuliginosa -

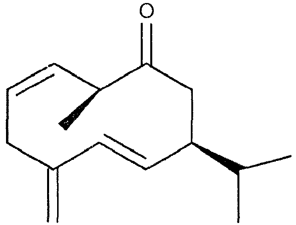

### - Alarm pheromone of aphid -

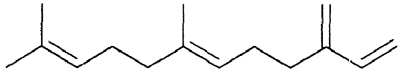

### - Aggregation pheromones of Dermatophagoides pteronyssinus -

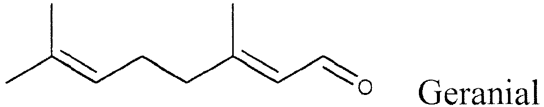

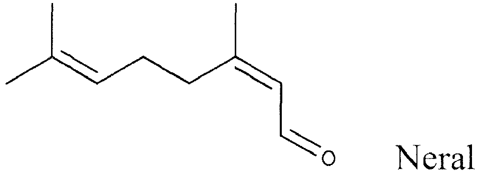

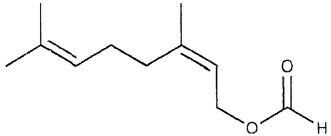

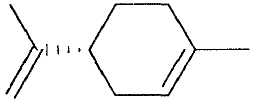

### - Sex pheromones of acarid mite of family Caloglyphini -

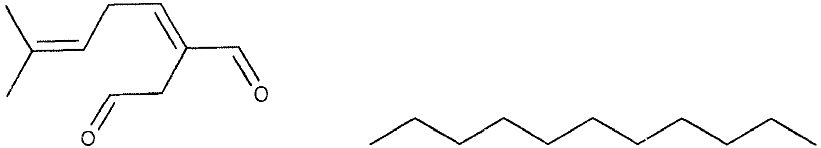

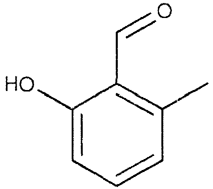

By using the detection method according to the embodiment of the invention, for example, geranial, which is a pheromone of Dermatophagoides pteronyssinus that is said to be most abundant among the mites settling in residences, and neral, which is a geometrical isomer of geranial, can be detected with high sensitivity.

### [Gas sensor for insect detection]

A gas sensor for insect detection according to the embodiment of the invention is a gas sensor suitable for being used in the detection method according to the embodiment of the invention as described above. That is, the gas sensor for insect detection according to the embodiment of the invention is a gas sensor selected from a resonant sensor, an electrical resistance sensor and an FET sensor, and the SP value of the gas adsorbing material constituting the gas adsorption membrane carried by the gas sensor is 13.9 to 23.9. This SP value is more preferably 15.9 to 20.9. A gas sensor including such a gas adsorption membrane is suitable particularly for the detection of geranial, neral, and the like emitted by mites.

A preferred embodiment of the gas sensor for insect detection according to the invention is the same as the embodiment of the as sensor explained in connection with the detection method according to the invention as described above.

### [Gas sensor array for insect detection]

A gas sensor array for insect detection according to the embodiment of the invention has a plurality of the above-described gas sensors for insect detection according to the invention integrated together. The gas adsorption membranes of the plurality of gas sensors for insect detection constituting the gas sensor array for insect detection according to the embodiment of the invention have mutually different gas adsorptiveness.

That is, the gas sensor array for insect detection according to the embodiment of the invention is such that the gas responsiveness of a plurality of gas sensors for insect detection constituting the array is mutually different. By using the gas sensor array for insect detection according to the embodiment of the invention, the detection of insects based on the signal patterns produced by a plurality of gas sensors is enabled as described above.

In regard to the gas sensor array for insect detection according to the embodiment of the invention, as long as a plurality of the gas sensors for insect detection according to the invention (the SP value of the gas adsorbing material constituting the gas adsorption membrane is 13.9 to 23.9) are integrated, some of the gas sensors constituting the array may be such that the SP value of the gas adsorbing material constituting the gas adsorption membrane is in the range of 13.9 to 23.9.

The number of the as sensors constituting the gas sensor array for insect detection according to the embodiment of the invention is preferably 2 to 100, more preferably 2 to 50, and even more preferably 4 to 30.

The gas sensors constituting the gas sensor array for insect detection according to the embodiment of the invention may include two or more kinds of sensors selected from a resonant sensor, an electrical resistance sensor, and an FET sensor; however, usually, all of the gas sensors constituting the gas sensor array are resonant sensors, all of them are electrical resistance sensors, or all of them are FET sensors. More preferably, all of the gas sensors constituting the gas sensor array for insect detection are resonant sensors.

The gas sensor array for insect detection according to the embodiment of the invention is also suitable to be used for the detection method according to the embodiment of the invention.

### [Electric machine product]

An electric machine product according to the embodiment of the invention is an electric machine product in which the gas sensor for insect detection according to the embodiment of the invention or the gas sensor array for insect detection according to the embodiment of the invention is mounted, and which has a function of detecting an insect by detecting an intrinsic gas emitted by the insect. Examples of such an electric machine product include a vacuum cleaner, an air cleaner, and an air conditioner. In a case in which the target of detection by the gas sensor to be mounted is mite, it is preferable that the electric machine product according to the embodiment of the invention is a vacuum cleaner. Thereby, the vacuum cleaner can be operated while the presence of mites is monitored, and thus, it is made possible to comprehend the usual state of cleaning, or to efficiently suction and remove mites.

The present invention will be described in detail based on Examples; however, the invention is not limited to these embodiments.

### Examples

### [Production Example 1-1] Production of electric resistance sensor 1

On a quartz glass having a size of 2.5 cm in length × 2.5 cm in width, two Ir electrodes were formed as films by sputtering, with the distance between the electrodes being set to 2 mm, and thus an electrode substrate was produced. Subsequently, the electrode substrate was cut out, and a strip having a size of 2.5 cm in length × 0.7 cm in width was produced. Thus, a strip having a distance between electrodes of 2 mm was obtained.

A coating liquid was obtained by dissolving 160 mg of polyvinyl stearate (hereinafter, referred to as "PVS") as a gas adsorbing material in 20 ml of toluene, subsequently adding 40 mg of carbon black as an electroconductive material to the solution, and ultrasonically treating the mixture for about 10 minutes.

0.1 ml of this coating liquid was dropped on the strip so as to connect between the two electrodes, and then the strip was heated for 120 minutes at 120°C using a hot plate. Thus, an electrical resistance sensor 1 having a gas detection unit on the substrate was obtained.

### [Production Example 1-2] Production of electrical resistance sensor 2

An electrical resistance sensor 2 was produced in the same manner as in Production Example 1-1, except that with regard to Production Example 1-1 described above, polybutyl methacrylate (hereinafter, referred to as "PBMA") was used as the gas adsorbing material instead of PVS.

### [Production Example 1-3] Production of electrical resistance sensor 3

An electrical resistance sensor 3 was produced in the same manner as in Production Example 1-1, except that with regard to Production Example 1-1 described above, polyethylene vinyl acetate (hereinafter, referred to as "PEVA") was used as the gas adsorbing material instead of PVS.

### [Production Example 1-4] Production of electrical resistance sensor 4

An electrical resistance sensor 4 was produced in the same manner as in Production Example 1-1, except that with regard to Production Example 1-1 described above, polycaprolactone (hereinafter, referred to as "PCL") was used as the gas adsorbing material instead of PVS.

### [Production Example 1-5] Production of electrical resistance sensor 5

An electrical resistance sensor 5 was produced in the same manner as in Production Example 1-1, except that with regard to Production Example 1-1 described above, polyvinylpyrrolidone (hereinafter, referred to as "PVP") was used as the gas adsorbing material instead of PVS, and tetrahydrofuran was used instead of toluene.

### [Test Example 1] Detection of mite pheromone

The various sensors produced in various Production Examples described above were exposed to a nitrogen gas atmosphere containing 1 ppm of citral (manufactured by Wako Pure Chemical Industries, Ltd., a mixed gas of geranial and neral), and the electrical resistance values were measured using an oscilloscope. The measured values thus obtained were evaluated by applying to the following evaluation criteria.

### <Evaluation criteria for electrical resistance value>

A: The electrical resistance value is 1,000 to 10,000 kΩ.
B: The electrical resistance value is 100 to 999 kΩ.
C: The electrical resistance value is 10 to 99 kΩ.
D: The electrical resistance value is 1 to 9 kΩ.
E: An increase in the electrical resistance value is not recognized (the electrical resistance value is less than 1 kQ).

The results are presented in the following table.

**[Table 1]**

| | Gas adsorbing material | Evaluation |
|---|---|---|
| Electrical resistance sensor 1 | PVS | C |
| Electrical resistance sensor 2 | PBMA | A |
| Electrical resistance sensor 3 | PEVA | A |
| Electrical resistance sensor 4 | PCL | B |
| Electrical resistance sensor 5 | PVP | E |

As shown in Table 1, it is understood that pheromones of an insect can be detected by means of an electrical resistance sensor. It is also understood that the sensitivity of the sensor can be regulated by changing the gas adsorbing material used for the electrical resistance sensor.

### [Production Example 2-1] Production of resonant sensor 1

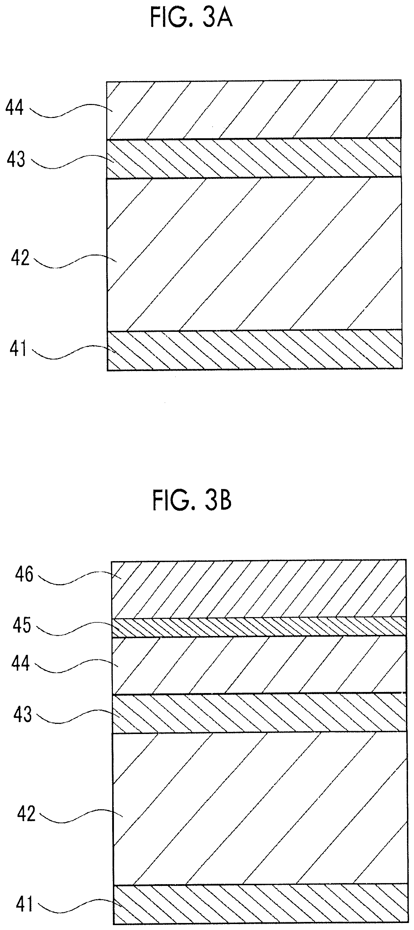

A resonant gas sensor (cantilever type) was produced according to the production flow schematically illustrated in Fig. 3A to Fig. 3S. The details will be explained.

As a substrate, a Silicon on Insulator (SOI) substrate as illustrated in Fig. 3A was used. This substrate has a laminated structure having a SiO₂ film (41, thickness 1 µm), a Si film (42, thickness 400 µm), a SiO₂ film (43, thickness 1 µm), and a Si film (44, thickness 15 µm) in order from the bottom in Fig. 3A.

On the substrate, a Ti film (thickness 20 nm) and an Ir film (thickness 100 nm) were formed successively as a first electrode (45, lower electrode) by a DC sputtering method. Next, a PZTN film (46, thickness 3 µm) was formed on the Ir film by an RF sputtering method (Fig. 3B). The film forming conditions will be shown below.

### - First electrode film forming conditions -

Substrate heating temperature: about 350°C
Input power: DC 500 W
Gas: Ar gas
Film forming pressure: 0.4 Pa

### - PZTN film forming conditions -

Substrate heating temperature: about 500°C
Input power: RF 1 kW
Gas: Ar gas : Oxygen (volume ratio 10:1)
Film forming pressure: 0.35 Pa

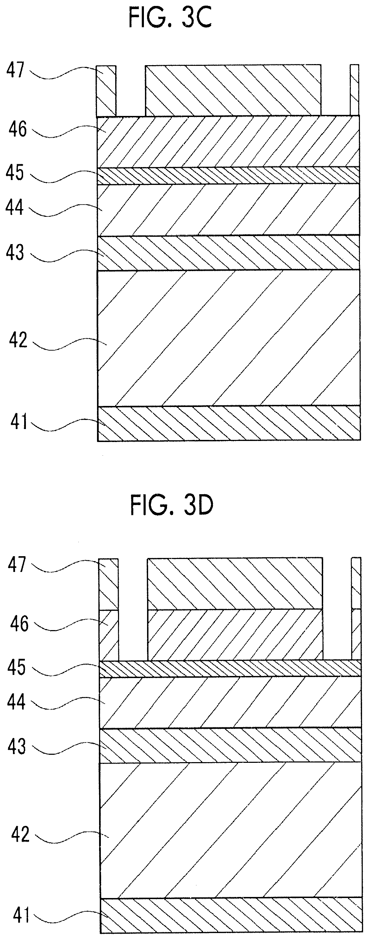

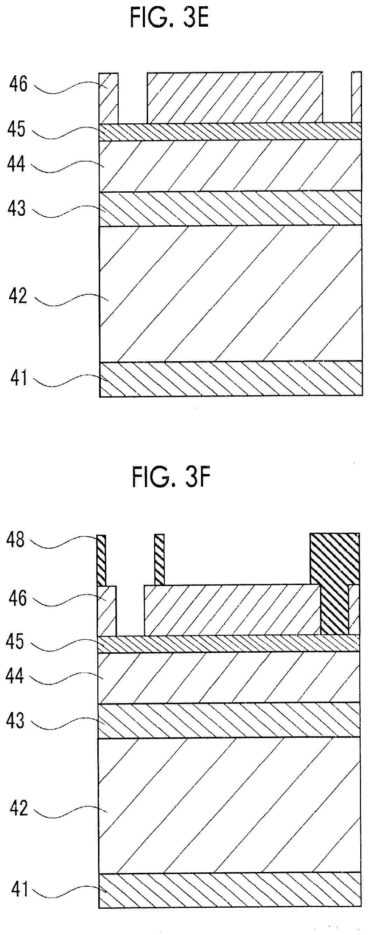

A resist (47) was formed for the patterning of the PZTN film [photoresist coating (AZ-1500, manufactured by Merck & Co., Inc.) → drying → exposure and development → baking, Fig. 2C]. Next, the PZTN film was wet-etched (Fig. 2 → drying → exposure and development → baking, Fig. 3C). Next, the PZTN film was wet-etched (Fig. 3D), and the resist (47) was removed using a resist stripping liquid (MS2001, manufactured by Fujifilm Corporation) (Fig. 3E).

A resist (48) for forming a contact with the lower electrode and for forming an upper electrode was formed [photoresist coating (AZ-5214, manufactured by Merck & Co., Inc.) → drying → exposure → baking → negative-positive reversal exposure → development → drying, Fig. 3F].

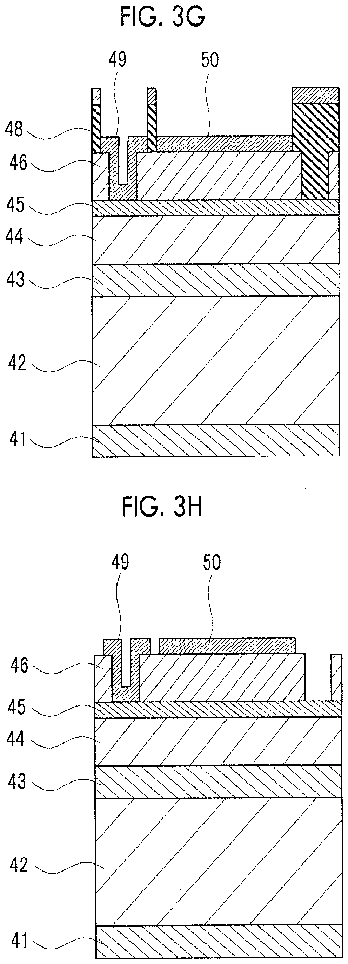

A Ti film (thickness 20 nm) and an Au film (thickness 100 nm) were formed successively as a contact (49) with the lower electrode and as a second electrode (50, upper electrode) by a DC sputtering method. The film forming conditions will be shown below (Fig. 3G).

### - Film forming conditions for contact with lower electrode and second electrode -

Substrate heating temperature: room temperature
Input power: DC 500 W
Gas: Ar gas
Film forming pressure: 0.4 Pa

Next, the resist (48) was removed using a resist stripping liquid (MS2001, manufactured by Fujifilm Corporation), a contact (49) with the lower electrode was formed, and an upper electrode (50) was formed (Fig. 3H).

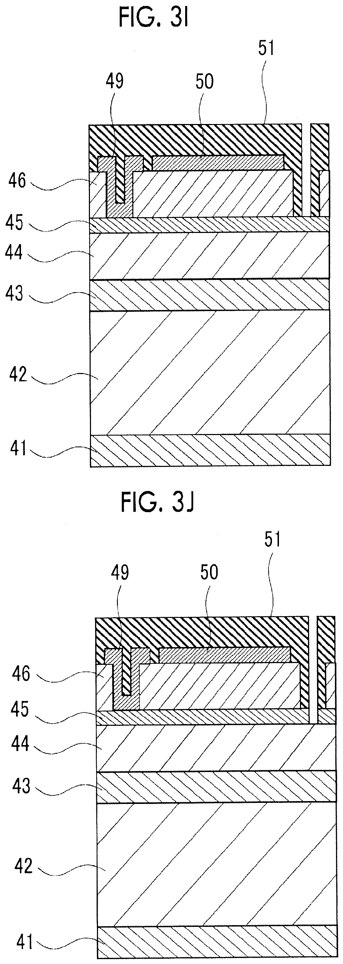

A resist (51) for patterning of the lower electrode and for patterning of the substrate surface layer (15 µm Si film) was formed [photoresist coating (AZ-1500, manufactured by Merck & Co., Inc.) → drying → exposure → development → baking, Fig. 3I].

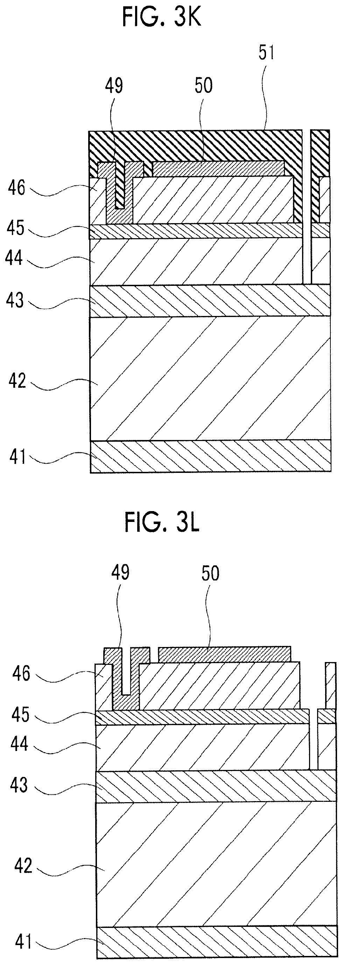

The lower electrode was removed by drying etching, subsequently the Si film was etched, and then the resist (51) was removed with a resist stripping liquid (MS2001, manufactured by Fujifilm Corporation) (Fig. 3J, Fig. 3K, and Fig. 3L).

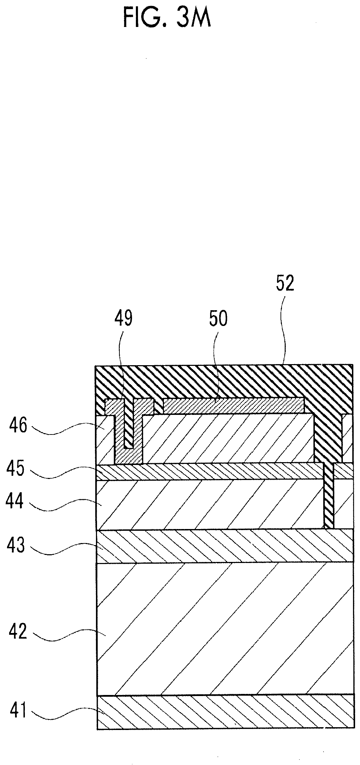

For the purpose of protection in the subsequent step, a resist (52) was formed on the upper electrode side [photoresist coating (AZ-10XT, manufactured by Merck & Co., Inc.) → drying → baking, Fig. 3M].

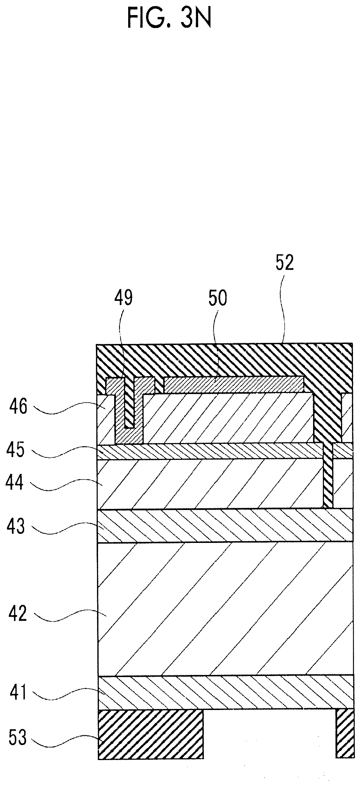

A resist (53) for patterning of the lower surface of the substrate was formed [photoresist coating (AZ-3100, manufactured by Merck & Co., Inc.) → drying → exposure → development → baking, Fig. 3N].

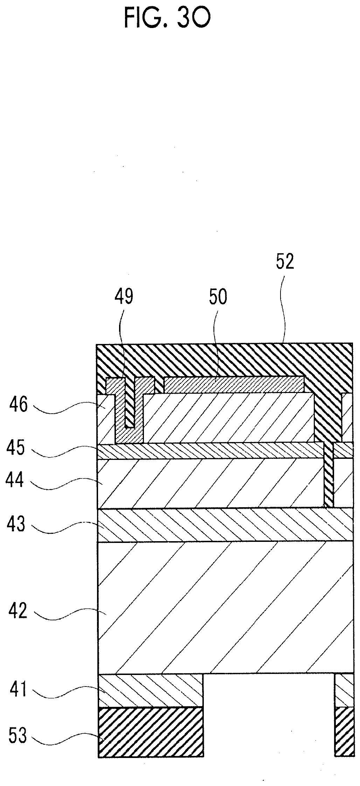

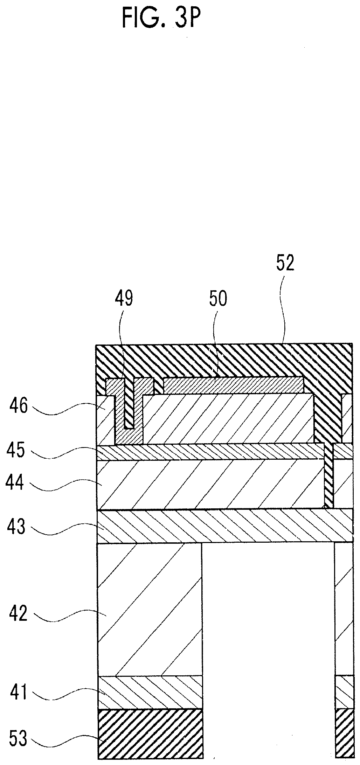

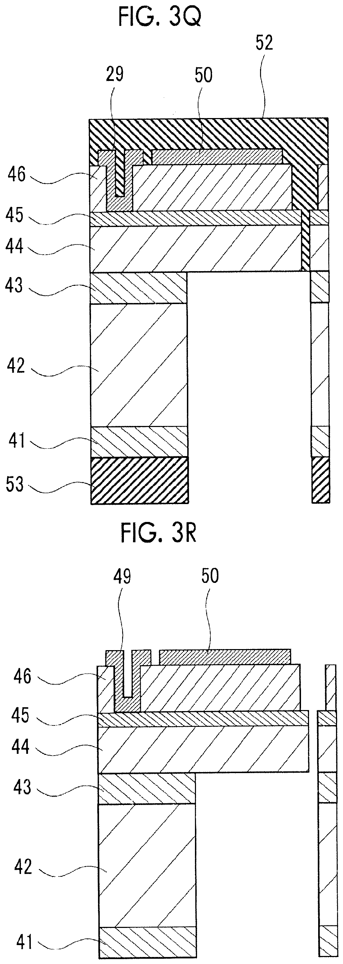

From the lower surface of the substrate, the 1-µm SiO₂ film (41), the 400-µm Si film (42), and the 1-µm SiO₂ film (43) were removed by drying etching, and all the resists were removed using a resist stripping liquid (MS2001, manufactured by Fujifilm Corporation) (Fig. 3O, Fig. 3P, Fig. 3Q, and Fig. 3R). A structure thus obtained, which is shown on the left-hand side in Fig. 3R, will be referred to as element precursor.

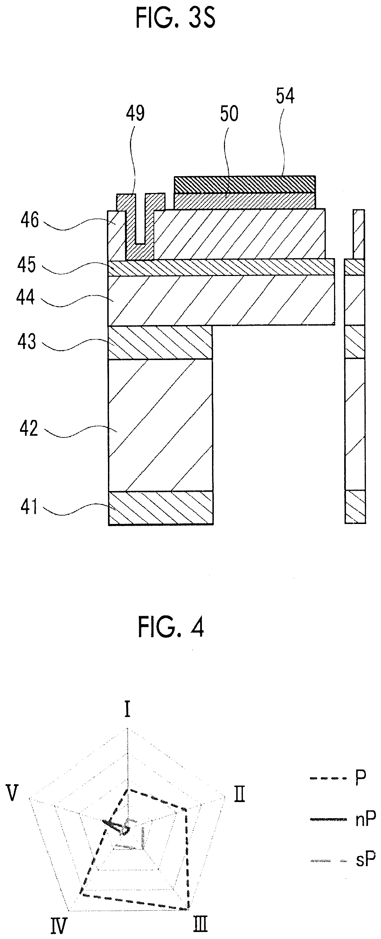

Next, a gas adsorption membrane as shown in Fig. 3S was formed on the upper electrode of the element precursor as follows.

### <Preparation of coating liquid for gas adsorption membrane formation>

PVS as a gas adsorbing material was dissolved in toluene, and a coating liquid containing 1% by mass of PVS was prepared.

### <Formation of gas adsorption membrane>

The coating liquid was introduced into a cartridge (type: DMCLCP-11610) of an inkjet printer (type: DMP-2831, manufactured by Fujifilm Corporation).

The element precursor was subjected to a UV cleaner treatment for 5 minutes using an apparatus manufactured by Jelight Co., Inc. (Model: 144AX-100). Immediately after this treatment, the coating liquid was jetted using the above-mentioned inkjet printer (jetting 6 times at a pitch of 50 µm, 10 pL discharged in one time of jetting, jetting speed: about 7 m/s), and a coating film was formed.

In order to completely volatilize toluene, the coating film was dried for 2 hours at 120°C in a vacuum oven (VAC-100, manufactured by ESPEC Corp.).

In this manner, a resonant sensor 1 having a first electrode, a dielectric sensor, a second electrode, and a gas adsorption membrane formed in this order on a support, as shown on the left-hand side of Fig. 3S, was obtained.

### [Production Example 2-2] Production of resonant sensor 2

A resonant sensor 2 was produced in the same manner as in Production Example 2-1, except that with respect to Production Example 2-1, PBMA was used as the gas adsorbing material instead of PVS.

### [Production Example 2-3] Production of resonant sensor 3

A resonant sensor 3 was produced in the same manner as in Production Example 2-1, except that with respect to Production Example 2-1, PEVA was used as the gas adsorbing material instead of PVS.

### [Production Example 2-4] Production of resonant sensor 4

A resonant sensor 4 was produced in the same manner as in Production Example 2-1, except that with respect to Production Example 2-1, PCL was used as the gas adsorbing material instead of PVS.

### [Production Example 2-5] Production of resonant sensor 5

A resonant sensor 5 was produced in the same manner as in Production Example 2-1, except that with respect to Production Example 2-1, PVP was used as the gas adsorbing material instead of PVS, and tetrahydrofuran was used instead of toluene.

### [Test Example 2] Detection of mite pheromone using resonant sensor array - 1

Resonant sensors 1 to 5 were installed in a nitrogen gas atmosphere, and the sensors were resonance-driven by applying a voltage (alternating current voltage, 200 to 400 kHz scan 0.1 Vrms sinusoidal wave) between the first electrode and the second electrode. The resonance frequencies were measured. The resonance frequency of each sensor in this state will be referred to as reference frequency.

Next, in a room in which a carpet was laid, the resonant sensors 1 to 5 were left to stand at a height of 10 cm from the carpet, the sensors were resonance-driven by applying a voltage (alternating current voltage, 200 to 400 kHz scan 0.1 Vrms sinusoidal wave) between the first electrode and the second electrode, and the resonance frequencies were measured. These measured values will be referred to as measured frequencies.

For each of the resonant sensors, the value of subtracting the measured frequency from the reference frequency (signal intensity) was calculated.

### [Test Example 3] Detection of mite pheromone using resonant sensor array - 2

After Test Example 2, a portion of the carpet measuring about 1 m on each of four sides in the carpet-laid room where Test Example 2 was carried out was vacuumed with a vacuum cleaner for about 1 minute. The vacuum cleaner was used without a pipe joint. Furthermore, the vacuumed matter was trapped in a new dust removal bag.

Resonant sensors 1 to 5 were left to stand inside the dust removal bag that had trapped the vacuumed matter of the vacuum cleaner, and the value of subtracting the measured frequency from the reference frequency was calculated in the same manner as described above.

Furthermore, a portion of the floor measuring about 1 m on each of four sides in a room with flooring was vacuumed with a vacuum cleaner in the same manner as described above, and the gas inside the dust removal bag was detected using resonant sensors 1 to 5 in the same manner as described above.

The results are shown in Fig. 4. In Fig. 4, I represents the signal intensity of resonant sensor 1; II represents the signal intensity of resonant sensor 2; III represents the signal intensity of resonant sensor 3; IV represents the signal intensity of resonant sensor 4; and V represents the signal intensity of resonant sensor 5.

### [Reference Example 1] Detection of mites using commercially available mite checker

For the dust removal bag used for the measurement in Test Example 3 described above, the amount of mites was quantitatively determined using a commercially available mite checker (trade name: MITEY CHECKER for mite detection, manufactured by Sumika Enviroscience Co., Ltd.). As a result, it was found that the number of mites in the dust removal bag used for vacuuming the carpet was more than 350 mites/m², and the number of mites in the dust removal bag used for vacuuming the flooring was fewer than 10 mites/m² (mites undetected).

In Fig. 4, P represents the results obtained by measuring in the dust removal bag used for vacuuming the carpet (more than 350 mites/m²) in Test Example 3; nP represents the results obtained by measuring in the dust removal bag used for vacuuming the flooring (fewer than 10 mites/m²) in Test Example 3; and sP represents the results obtained by measuring at a height of 10 cm from the carpet in Test Example 2.

As shown in Fig. 4, in all of the measurement at a height of 10 cm from the carpet and measurement in a dust removal bags used for vacuuming the carpet (dust removal bags in which mites were trapped (concentrated)), it was found that gas was detected with different signal intensities for all of resonant sensors 1 to 5, and the signal patterns of the resonant sensor array in various measurements showed a similar shape.

In contrast, upon sensing of the dust removal bag used for vacuuming the flooring where no mites were found, the signals of the various resonant sensors were almost not detected, and the signal pattern of the sensor array also completely differed from the signal patterns obtained in the measurement at a height of 10 cm from the carpet or the measurement in the dust removal bag used for vacuuming the carpet.

The results described above imply that gas components emitted from insects and hang in the space can be detected by a gas sensor including a gas adsorption membrane, and thereby the insects can be detected.

The present invention has been explained together with embodiments thereof; however, the present invention is not intended to be limited to any details of the description unless otherwise specified, and it is to be understood that the invention should be broadly interpreted without departing from the spirit and scope of the invention as set forth in the appended claims.

The present patent application claims priority from JP2017-63606, filed in Japan on March 28, 2017, the disclosure of which is incorporated herein by reference in its entirety. Explanation of References
1: first electrode
2: dielectric material (piezoelectric material)
3: second electrode
4: gas adsorption membrane
10: transistor
20: substrate
22: gate electrode
24: gate insulating layer
26: semiconductor layer
28: source electrode
30: drain electrode
32: gas adsorption membrane
41: SiO₂ film
42: Si film
43: SiO₂ film
44: Si film
45: first electrode
46: PZTN film
47, 48, 51, 52, 53: resist
49: contact with lower electrode
50: second electrode
P: measurement in dust removal bag used for vacuuming carpet
nP: measurement in dust removal bag used for vacuuming flooring
sP: measurement at height of 10 cm from carpet
I: signal intensity of resonant sensor 1
II: signal intensity of resonant sensor 2
III: signal intensity of resonant sensor 3
IV: signal intensity of resonant sensor 4
V: signal intensity of resonant sensor 5

## Claims

1. An insect detection method, comprising detecting an intrinsic gas emitted by an insect using a gas sensor including a gas adsorption membrane, the gas sensor being selected from a resonant sensor, an electrical resistance sensor, and a field effect transistor sensor.

2. The insect detection method according to claim 1, wherein the dielectric material carried by the resonant sensor is a ceramic dielectric material or a quartz crystal.

3. The insect detection method according to claim 2, wherein the ceramic dielectric material is lead zirconate titanate, niobium-doped lead zirconate titanate, zinc oxide, or aluminum nitride.

4. The insect detection method according to any one of claims 1 to 3, wherein the method includes detecting an intrinsic gas emitted by an insect using a plurality of gas sensors, and the plurality of gas sensors have mutually different gas responsiveness.

5. The insect detection method according to claim 4, wherein an insect is detected on the basis of a pattern of gas detection signals by the plurality of gas sensors.

6. The insect detection method according to any one of claims 1 to 5, wherein the gas sensor is a resonant sensor.

7. The insect detection method according to any one of claims 1 to 6, wherein the insect is a mite, a cockroach, a termite, or an aphid.

8. The insect detection method according to claim 7, wherein the insect is a mite.

9. A gas sensor for insect detection, comprising a gas adsorption membrane, the gas sensor being selected from a resonant sensor, an electrical resistance sensor, and a field effect transistor sensor,
wherein the gas adsorbing material constituting the gas adsorption membrane of the gas sensor has a solubility parameter of 13.9 to 23.9.

10. A gas sensor array for insect detection, comprising a plurality of the gas sensors for insect detection according to claim 9 integrated together, wherein this plurality of gas sensors have mutually different gas responsiveness.

11. An electric machine product, comprising the gas sensor for insect detection according to claim 9 or the gas sensor array for insect detection according to claim 10, mounted therein.

12. The electric machine product according to claim 11, wherein the electric machine product is a vacuum cleaner.
